(19) Europäisches Patentamt
European Patent Office
Office européen des brevets

(11) Veröffentlichungsnummer : **0 143 147**
**B1**

(12) **EUROPÄISCHE PATENTSCHRIFT**

(45) Veröffentlichungstag der Patentschrift : **09.09.87**

(21) Anmeldenummer : **84103403.6**

(22) Anmeldetag : **28.03.84**

(51) Int. Cl.[4] : **C 07 C101/08**, C 07 D207/16, C 07 D209/20, C 07 C101/04, C 07 C101/22, C 07 C149/247, C 07 D277/06, C 07 C 99/12, C 07 B 57/00

# (54) Verfahren zur dünnschichtchromatographischen Trennung von Enantiomeren.

(30) Priorität : **05.08.83 DE 3328348**

(43) Veröffentlichungstag der Anmeldung : **05.06.85 Patentblatt 85/23**

(45) Bekanntmachung des Hinweises auf die Patenterteilung : **09.09.87 Patentblatt 87/37**

(84) Benannte Vertragsstaaten : **AT BE CH DE FR GB LI NL**

(56) Entgegenhaltungen :
**ANGEWANDTE CHEMIE, Band 96, Heft 3, 1984, Seiten 514-515, Weinheim-Bergstrasse; K. GÜNTHER et al.: "Dünnschichtchromatographische Enantiomerentrennung mittels Ligandenaustausch"**
**CHEMICAL ABSTRACTS, Band 98, Nr. 10, 7. März 1983, Seite 388, Spalte 1, Abstract Nr. 78714d, Columbus, Ohio, US; P. ROUMELIOTIS et al.: "High-performance ligand-exchange chromatography of alpha-amino acid enantiomers. Studies on monomerically bonded 3-(L-prolyl)- and 3-(L-hydroxyprolyl) propyl silicas"**
**CHEMICAL ABSTRACTS, Band 94, Nr. 10, 9. März 1981, Seite 426, Spalte 1, Abstract Nr. 72109f, Columbus, Ohio, US; J. BOUE et al.: "Direct resolution of alpha-amino acid enantiomers by ligand exchange: stereoselection mechanism on silica packings coated with a chiral polymer"**
**CHEMICAL ABSTRACTS, Band 94, Nr. 12, 23. März 1981, Seite 788, Spalte 2, Abstract Nr. 95401j, Columbus, Ohio, US; V.A. DAVANKOV et al.: "Ligand-exchange chromatography. 13. Separation of unmodified alpha-amino acid enantiomers by reverse phase HPLC"**

(73) Patentinhaber : **Degussa Aktiengesellschaft**
**Weissfrauenstrasse 9**
**D-6000 Frankfurt am Main 1 (DE)**

(72) Erfinder : **Günther, Kurt, Dr. Dipl.-Chem.**
**Goethestrasse 4**
**D-6455 Erlensee (DE)**
Erfinder : **Martens, Jürgen, Dr. Dipl.-Chem.**
**Hochstrasse 10**
**D-8755 Alzenau (DE)**
Erfinder : **Schickedanz, Maren**
**Frankfurterstrasse 32 A**
**D-6057 Dietenbach (DE)**

**EP 0 143 147 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

CHEMICAL ABSTRACTS, Band 81, Nr. 8, 26. August 1974, Seite 325, Spalte 2, Abstract Nr. 42220z, Columbus, Ohio, US; F. KARCZYNSKI et al.: "Complex compounds of amino acids. III. Chromatographic study on alpha-amino acid complexes of Cu(II)"
SOVIET INVENTIONS ILLUSTRATED, Sektion Chemical, Woche E/43, 8. Dezember 1982, Zusammenfassungsnr. 92121 B 05, Derwent Publications Ltd., London, GB

**Beschreibung**

Die Erfindung betrifft ein Verfahren zur dünnschichtchromatographischen Trennung von Enantiomeren der allgemeinen Formel

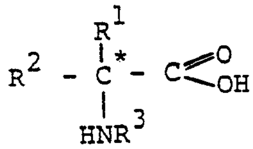 (I)

in der C* ein Asymmetriezentrum darstellt und die Substituenten $R^1$, $R^2$ und $R^3$ solche Bedeutungen haben, daß sie das Strukturelement

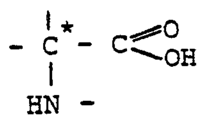

zu einer an sich bekannten α-Amino- oder α-Iminocarbonsäure oder einem Derivat einer solchen Carbonsäure ergänzen, welche bzw. welches keine freien Mercaptogruppen aufweist, mittels Ligandenaustausch an einer chiralen stationären Phase.

Aus Chemical Abstracts, Vol. 81 (1974), Referat 42220z, ist es bereits bekannt, Cu-(II)-Komplexe verschiedener Aminosäuren durch Dünnschichtchromatographie in wäßrigen Alkoholen zu trennen. Bei Einsatz von Lösungen, die zwei verschiedene Aminosäuren enthalten, wird nur mit einer dieser beiden Aminosäuren ein Cu-(II)-Komplex gebildet.

Es ist ferner auch bereits bekannt, Enantiomere durch Hochdruck-Flüssigkeitschromatographie (HPLC) an einer chiralen stationären Phase zu trennen, wobei bevorzugt die Methode der Ligandenaustauschchromatographie angewandt wird [W. Lindner, Chimia 35, 294 (1981)]. Im einzelnen ist in Chemical Abstracts, Vol. 94 (1981), Referat 94 : 72109 f, die Trennung von Aminosäure-Enantiomeren auf Kieselgel, an dem ein chirales Polymeres auf der Grundlage von einem linearen Polyacrylamid und L-Prolin adsorbiert ist, beschrieben. In Chemical Abstracts, Vol. 94 (1981), Referat 94 : 95401j, wird eine neuartige chirale Phase für die Trennung von Aminosäure-Enantiomeren vorgestellt, bei der ein hydrophobiertes Kieselgel mit N-(längerkettig) Alkyl-L-hydroxyprolin überzogen ist. In Soviet Inventions Illustrated, Sektion Chemical, Woche E/43 (1982), wird die Trennung von Aminosäure-Enantiomeren an einer stationären Phase aus einem Kieselgel mit Asymmetriezentren, das einen chemisch gebundenen Kohlenwasserstoff enthält, geschildert. Das Laufmittel muß geringe Mengen an 1-Benzyl-prolin enthalten.

In Chemical Abstracts, Vol. 98 (1983), Referat 98 : 78714d, schließlich ist die Trennung von Aminosäure-Enantiomeren an einem hydrophobierten Kieselgel beschrieben, das Cu-(II)-Komplexe von 3-(L-Prolyl)-bzw. 3-(L-Hydroxyprolyl)-propyl-gruppen aufweist. Alle diese bekannten HPLC-Trennverfahren haben jedoch den Nachteil, daß sie relativ aufwendige und störanfällige Apparate erfordern, und daß für die Vorbereitung und Durchführung einer Trennung eine beträchtliche Zeit aufgewendet werden muß. Sie sind daher für Routineanalysen und insbesondere für Betriebskontrollen weniger geeignet.

Der Erfindung lag daher die Aufgabe zugrunde, die Trennung von Enantiomeren der allgemeinen Formel (I) wesentlich zu vereinfachen und insbesondere eine für Betriebskontrollen, beispielsweise Reinheitsprüfungen, geeignete einfache und mit relativ geringem Zeitaufwand durchführbare Methode zu schaffen.

Das erfindungsgemäße Verfahren ist nun dadurch gekennzeichnet, daß man die Trennung auf einer an sich bekannten, mit durch ein Silanderivat hydrophobiertem Kieselgel belegten Dünnschichtchromatographie-Platte vornimmt, die zusätzlich mit einer ionischen Verbindung eines zweiwertigen Übergangsmetalls und einem chiralen Selektor imprägniert ist, und als Laufmittel ein ternäres Gemisch aus einem mit Wasser mischbaren Alkanol, Wasser und Acetonitril oder ein quaternäres Gemisch aus einem mit Wasser mischbaren Alkanol, Wasser, Acetonitril und einem mit Wasser mischbaren cyclischen Ether einsetzt.

Als chirale stationäre Phase für die Dünnschichtchromatographie (DC) dient eine an sich bekannte, mit hydrophobiertem Kieselgel belegte DC-Platte. Solche Platten mit Glas oder einer geeigneten Folie als Trägermaterial sind im Handel als sogenannte Reverse-Phase-Platten erhältlich. Das Kieselgel wurde bereits beim Hersteller durch chemische Bindung oder durch physikalische Adsorption einer hydrophoben Substanz hydrophobiert. Für das erfindungsgemäße Verfahren besonders geeignete Reverse-Phase-Platten sind solche vom Typ RP 2, RP 8, RP 12 und vorzugsweise RP 18. In diesen Bezeichnungen bedeuten die Zahlen, daß das Kieselgel mit einem Methyl-(2), Octyl-(8), Dodecyl-(12) bzw. Octadecylsilan-Derivat (18) hydrophobiert ist. Geeignete, für die Hydrophobierung zu verwendende Silan-Derivate sind beispielsweise die entsprechenden Trichlorsilane, Dichlor-methyl-silane oder Dimethoxy-methyl-silane.

Die beim erfindungsgemäßen Verfahren zu verwendenden DC-Platten müssen zusätzlich mit einer

ionischen Verbindung eines zweiwertigen Übergangsmetalls und einem chiralen Selektor imprägniert sein.

Geeignete Übergangsmetall-Verbindungen sind solche, welche die Ionen $Ni^{++}$, $Zn^{++}$, $Cd^{++}$, $Hg^{++}$, $Co^{++}$ oder insbesondere $Cu^{++}$ enthalten.

Geeignete chirale Selektoren sind grundsätzlich alle für die Ligandenaustauschchromatographie mittels HPLC üblicherweise verwendeten Verbindungen, beispielsweise N-Heptyl-L-hydroxyprolin, N-Decyl-L-hydroxypyrolin, N-Hexadecyl-L-hydroxyprolin oder die in [Chimia, 35, 294-307, (1981)] genannten chiralen Selektoren.

Besonders bevorzugte chirale Selektoren sind jedoch optisch aktive Prolin-Derviate der allgemeinen Formel

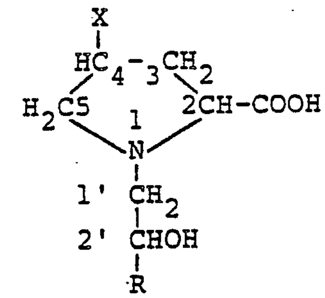

(II)

in der X Wasserstoff oder eine Hydroxygruppe und R einen unsubstituierten oder durch niedere Alkylgruppen substituierten Phenylrest oder einen geradkettigen oder verzweigten Alkylrest mit 1 bis 20 Kohlenstoffatomen, vorzugsweise 6 bis 16 Kohlenstoffatomen, insbesondere 10 bis 12 Kohlenstoffatomen, bedeuten. Die Prolin-Derivate der allgemeinen Formel (II) sind in jedem Falle sterisch einheitlich hinsichtlich des asymmetrischen Kohlenstoffatoms in 2-Stellung, im Falle X = OH auch hinsichtlich des asymmetrischen Kohlenstoffatoms in 4-Stellung. Die absolute Konfiguration des asymmetrischen Kohlenstoffatoms in 2'-Stellung der Seitenkette muß dagegen nicht einheitlich sein. Das heißt, daß das Prolin-Derivat der allgemeinen Formel (II) auch als ein Gemisch zweier Diastereomerer eingesetzt werden kann. Die Prolin-Derivate der allgemeinen Formel (II) und ein Verfahren zu ihrer Herstellung aus enantiomerenreinem Prolin bzw. 4-Hydroxyprolin und entsprechenden Epoxiden sind in der DE-OS 31 43 726 ausführlich beschrieben.

Die beim erfindungsgemäßen Verfahren zu verwendenden, mit durch ein Silanderivat hydrophobiertem Kieselgel belegten und zusätzlich mit einer ionischen Verbindung eines zweiwertigen Übergangsmetalls und einem chiralen Selektor imprägnierten DC-Platten lassen sich beispielsweise wie folgt herstellen :

Man geht aus von einer der oben beschriebenen käuflichen Reverse-Phase-Platten. Diese Platte wird zunächst in eine Lösung einer geeigneten Übergangsmetallverbindung, beispielsweise Kupfer-II-acetat, in einem Gemisch aus 1 Volumenteil Methanol und 9 Volumenteilen Wasser eingetaucht. Die zweckmäßigen Konzentrationen der Übergangsmetallverbindung in dieser Lösung liegen in dem Bereich zwischen 0,01 und 1,0 Gewichtsprozent, vorzugsweise zwischen 0,1 und 0,5 Gewichtsprozent. Als Tauchzeit reicht im allgemeinen etwa 1 Minute völlig aus. Man läßt die Platte dann kurz abtropfen und trocknet sie bei einer Temperatur zwischen 90 und 150 °C, vorzugsweise zwischen 100 und 120 °C. Anschließend wird die Platte in eine methanolische Lösung des chiralen Selektors eingetaucht. Die zweckmäßigen Konzentrationen des Selektors in dieser Lösung liegen in dem Bereich zwischen 0,1 und 2,5 Gewichtsprozent, vorzugsweise zwischen 0,5 und 1,5 Gewichtsprozent, insbesondere zwischen 0,7 und 1,1 Gewichtsprozent. Als Tauchzeit reicht wiederum im allgemeinen etwa 1 Minute völlig aus. Man läßt die Platte wieder kurz abtropfen und bei einer Temperatur zwischen 20 und 30 °C an der Luft trocknen. Die so präparierte Platte ist für das erfindungsgemäße Verfahren direkt verwendbar und einige Monate lagerstabil.

Alternativ kann die Herstellung geeigneter DC-Platten auch dadurch erfolgen, daß man eine Aufschlämmung eines z. B. nach Journal of Chromatography 154, 68 (1978) hergestellten hydrophoben Kieselgels in einem Gemisch aus Ethanol und Wasser mit der ionischen Verbindung des zweiwertigen Übergangsmetalls und dem chiralen Selektor versetzt und nach Zusatz eines Bindemittels, beispielsweise Calciumsulfat, eine Trägerplatte aus Glas oder einer geeigneten Folie damit beschichtet.

Zur Durchführung der Trennung trägt man auf den Startpunkt wie üblich etwa 2 µl einer etwa 1 gewichtsprozentigen Lösung der zu untersuchenden Probe auf und entwickelt in üblicher Weise mit einem geeigneten Laufmittel in einer DC-Kammer.

Für das erfindungsgemäße Verfahren geeignete Laufmittel sind ternäre Gemische aus einem mit Wasser mischbaren Alkanol, Wasser und Acetonitril oder quaternäre Gemische, die zusätzlich zu den genannten Bestandteilen noch einen mit Wasser mischbaren cyclischen Ether enthalten. Als Alkanole sind Methanol, Ethanol, Propan-1-ol und Propan-2-ol geeignet, wobei das Methanol bevorzugt verwendet wird, als cyclische Ether 1,4-Dioxan und insbesondere Tetrahydrofuran. Die zweckmäßigsten Zusammensetzungen liegen in den ternären Gemischen im Bereich 25 bis 75 Volumenteile Alkanol, 25 bis 75

Volumenteile Wasser und 100 bis 300 Volumenteile Acetonitril, in den quaternären Gemischen im Bereich 30 bis 70 Volumenteile Alkanol, 30 bis 70 Volumenteile Wasser, 100 bis 150 Volumenteile Acetonitril und 50 bis 100 Volumenteile cyclischer Ether.

Die entwickelte Platte wird aus der DC-Kammer herausgenommen und mit Hilfe eines Föhns oder auf eine andere in der DC übliche Weise getrocknet. Die teilweise bereits im UV-Licht sichtbaren, den (R)- bzw. (S)-Enantiomeren zuzuordnenden Flecken können durch Besprühen mit einer Ninhydrin-Lösung oder einem anderen geeigneten Sprühreagenz, beispielsweise Lösungen von Schwefelsäure oder Jod, deutlich sichtbar gemacht werden.

Trägt man auf die DC-Platte parallel zu der zu untersuchenden Probe standardisierte Gemische der beiden zu trennenden Enantiomeren auf, so ist durch visuellen Vergleich der Fleckengrößen sogar eine halbquantitative Bestimmung der Zusammensetzung der zu untersuchenden Probe möglich. So lassen sich beispielsweise noch etwa 1 Gewichtsprozent D-Aminocarbonsäure in einer anracemisierten L-Aminocarbonsäure nachweisen.

Mittels des erfindungsgemäßen Verfahrens lassen sich Enantiomere der allgemeinen Formel (I) auf einfache Weise trennen. Besonders geeignet ist das erfindungsgemäße Verfahren für solche Verbindungen der allgemeinen Formel (I), für die einer der beiden Substituenten $R^1$ oder $R^2$ Wasserstoff bedeutet. Insbesondere ist es geeignet zur Trennung der Enantiomeren von Phenylalanin und dessen im Kern substituierten Derivaten, Tryptophan und dessen Derivaten, Tyrosin und O-substituierten Tyrosinen, Prolin, Isoleucin, Norleucin, Glutamin, 3-Thiazolidin-4-carbonsäure und deren in 5-Stellung substituierten Derivaten, S-substituierten Cystein-Derivaten und O-substituierten Serin-Derivaten. Ein weiteres Beispiel für eine Verbindung, deren Enantiomere durch das erfindungsgemäße Verfahren getrennt werden können, ist 3 Cyclopentyl-alanin.

. Die Erfindung soll durch die nachfolgenden Beispiele näher erläutert werden. Prozentangaben bedeuten in allen Fällen Gewichtsprozente.

## Beispiel 1

Eingesetzt wurde eine käufliche mit durch ein Octadecylsilanderivat hydrophobiertem Kieselgel beschichtete DC-Platte. Diese Platte wurde eine Minute lang in eine Lösung von 0,25 % Kupfer-II-acetat in einem Gemisch aus 1 Volumenteil Methanol und 9 Volumenteilen Wasser eingetaucht, kurz abtropfen gelassen und bei 110 °C getrocknet. Die so mit Kupfer-II-acetat imprägnierte Platte wurde anschließend eine Minute lang in eine 0,8 %-ige methanolische Lösung von (2S, 4R, 2'RS)-N-(2'-Hydroxydodecyl)-4-hydroxypyrolin eingetaucht, kurz abtropfen gelassen und bei Raumtemperatur an der Luft trocknen gelassen.

Es wurden eine 1 %-ige Lösung eines Phenylalanis unbekannter Enantiomerenzusammensetzung in einem Gemisch aus gleichen Volumenteilen Methanol und Wasser als zu untersuchende Probe, sowie zu Vergleichszwecken entsprechende Lösungen von racemischem (RS)-Phenylalanin und von reinem (R)- und (S)-Phenylalanin bereitet.

Jeweils 2 µl dieser Lösungen wurden auf die prärierte DC-Platte aufgetragen und mit einem Laufmittel aus Methanol, Wasser und Acetonitril im Volumenverhältnis 1 : 1 : 4 entwickelt.

Nachdem die Laufmittelfront nach 30 Minuten eine Höhe von 14 cm erreicht hatte, wurde das Lösungsmittelgemisch verdampfen gelassen, die Platte mit einer käuflichen 0,1 %-igen Ninhydrin-Lösung besprüht und 30 Minuten bei 120 °C getrocknet.

Das racemische (RS)-Phenylalanin zeigte zwei gleich große, deutlich voneinander getrennte violette Flecken mit den $R_f$-Werten 0,38 und 0,45, das reine (R)-Phenylalanin einen einzigen Fleck mit einem $R_f$-Wert von 0,38, das reine (S)-Phenylalanin einen einzigen Fleck mit einem $R_f$-Wert von 0,45 und die zu untersuchende Probe zwei verschieden große Flekken mit den $R_f$-Werten 0,38 und 0,45. Bei dieser Probe handelte es sich demnach um ein teilracemisiertes Phenylalanin.

## Beispiel 2

Das Beispiel 1 wurde wiederholt mit dem einzigen Unterschied, daß als Laufmittel ein Gemisch aus Ethanol, Wasser und Acetonitril im Volumenverhältnis 1 : 1 : 4 eingesetzt wurde. In diesem Falle führte das (R)-Phenylalanin zu Flecken mit dem $R_f$-Wert 0,37 und das (S)-Phenylalanin zu Flecken mit dem $R_f$-Wert von wiederum 0,45.

## Beispiel 3

Das Beispiel 1 wurde wiederholt mit dem einzigen Unterschied, daß als Laufmittel ein Gemisch aus Propan-2-ol, Wasser und Acetonitril im Volumenverhältnis 1 : 1 : 4 eingesetzt wurde. In diesem Falle führte wiederum das (R)-Phenylalanin zu Flecken mit dem $R_f$-Wert 0,37 und das (S)-Phenylalanin zu Flecken mit dem $R_f$-Wert 0,45.

## Beispiele 4 bis 9

Mittels der in Beispiel 1 beschriebenen präparierten DC-Platte und des dort verwendeten Laufmittels wurden verschiedene racemische in 4-Stellung substituierte Phenylalanine getrennt. Die Auswertung ergab folgendes :

| Beispiel | (RS)-Aminosäure | $R_f$-Werte |
|---|---|---|
| 4 | 4-Chlor-phenylalanin | 0,31 und 0,41 |
| 5 | 4-Brom-phenylalanin | 0,28 und 0,40 |
| 6 | 4-Jod-phenylalanin | 0,26 und 0,38 |
| 7 | 4-Nitro-phenylalanin | 0,41 und 0,48 |
| 8 | 4-Methoxy-phenylalanin | 0,38 und 0,46 |
| 9 | 3-Brom-phenylalanin | 0,32 und 0,41 |

Beispiel 10

Das Beispiel 1 wurde wiederholt mit dem Unterschied, daß als zu untersuchende Probe ein Tryptophan unbekannter Enantiomerenzusammensetzung und zu Vergleichszwecken (RS)-, (R)- und (S)-Tryptophan eingesetzt wurden. In diesem Falle führte das (R)-Tryptophan zu Flecken mit dem $R_f$-Wert 0,39 und das (S)-Tryptophan zu Flecken mit dem $R_f$-Wert 0,45.

Beispiele 11 bis 14

Mittels der in Beispiel 1 beschriebenen präparierten DC-Platte und des dort verwendeten Laufmittels wurden verschiedene racemische substituierte Tryptophane getrennt. Die Auswertung ergab folgendes :

| Beispiel | (RS)-Aminosäure | $R_f$-Werte |
|---|---|---|
| 11 | 5-Methyl-tryptophan | 0,36 und 0,43 |
| 12 | 6-Methyl-tryptophan | 0,36 und 0,45 |
| 13 | 7-Methyl-tryptophan | 0,35 und 0,44 |
| 14 | 5-Methoxy-tryptophan | 0,41 und 0,48 |

Beispiel 15

Das Beispiel 1 wurde wiederholt mit dem Unterschied, daß als zu untersuchende Probe ein Tyrosin unbekannter Enantiomerenzusammensetzung und zu Vergleichszwecken (RS)-, (R)- und (S)-Tyrosin eingesetzt und als Laufmittel ein Gemisch aus Methanol, Wasser und Acetonitril im Volumenverhältnis 1 : 1 : 0,6 verwendet wurden. In diesem Falle führte das (R)-Tyrosin zu Flecken mit dem $R_f$-Wert 0,34 und das (S)-Tyrosin zu Flecken mit dem $R_f$-Wert 0,26.

Beispiel 16

Mittels der in Beispiel 1 beschriebenen präparierten DC-Platte und des dort verwendeten Laufmittels wurde racemisches O-Benzyl-tyrosin getrennt. Die $R_f$-Werte betrugen 0,26 und 0,38.

Beispiel 17

Das Beispiel 15 wurde wiederholt mit dem Unterschied, daß als zu untersuchende Probe ein Prolin unbekannter Enantiomerenzusammensetzung und zu Vergleichszwecken (RS)-, (R)- und (S)-Prolin eingesetzt wurden. In diesem Falle führte das (R)-Prolin zu Flecken mit dem $R_f$-Wert 0,40 und das (S)-Prolin zu Flecken mit dem $R_f$-Wert 0,59.

Beispiel 18

Das Beispiel 1 wurde wiederholt mit dem Unterschied, daß als zu untersuchende Probe ein Isoleucin unbekannter Enantiomerenzusammensetzung und zu Vergleichszwecken (RS)-, (R)- und (S)-Isoleucin eingesetzt wurden. Alle eingesetzten Proben waren frei von allo-Isoleucin. In diesem Falle führte die (2S, 3S)-2-Amino-3-methyl-n-valeriansäure zu Flecken mit dem $R_f$-Wert 0,44 und die (2R, 3R)-2-Amino-3-methyl-n-valeriansäure zu Flecken mit dem $R_f$-Wert 0,37.

Beispiel 19

Mittels der in Beispiel 1 beschriebenen präparierten DC-Platte und des dort verwendeten Laufmittels wurde racemisches Norleucin getrennt. Die $R_f$-Werte betrugen 0,33 und 0,42.

Beispiel 20

Das Beispiel 1 wurde wiederholt mit dem Unterschied, daß als zu untersuchende Probe ein Glutamin unbekannter Enantiomerenzusammensetzung und zu Vergleichszwecken (RS)-, (R)- und (S)-Glutamin eingesetzt wurden und daß die verwendete DC-Platte anstatt mit (2S, 4R, 2'RS)-N-(2'-Hydroxydocecyl)-4-hydroxyprolin mit (2S, 2'RS)-N-(2'-Hydroxytetradecyl)-prolin als chiraler Selektor imprägniert war.

Nachdem die Laufmittelfront nach 40 Minuten eine Höhe von 14 cm erreicht hatte, wurde das Lösungsmittelgemisch verdampfen gelassen, die Platte mit einer käuflichen 0,1 %-igen Ninhydrin-Lösung besprüht und 45 Minuten bei 115 °C getrocknet.

Das racemische (RS)-Glutamin zeigte zwei gleich große, deutlich voneinander getrennte Flecken mit den $R_f$-Werten 0,19 und 0,35, das reine (R)-Glutamin einen einzigen Fleck mit einem $R_f$-Wert von 0,35, das reine (S)-Glutamin einen einzigen Fleck mit einem $R_f$-Wert von 0,19 und die zu untersuchende Probe zwei verschieden große Flecken mit den $R_f$-Werten 0,19 und 0,35. In dieser Probe lag also ein teilracemisiertes Glutamin vor.

Beispiel 21

Es wurde wie in Beispiel 20 verfahren mit dem Unterschied, daß die verwendete DC-Platte anstatt mit (2S, 2'RS)- mit (2R, 2'RS)-N-(2'-Hydroxytetradecyl)-prolin als chiraler Selektor imprägniert war. In diesem Falle führte das (R)-Glutamin zu Flecken mit dem $R_f$-Wert 0,19 und das (S)-Glutamin zu Flecken mit dem $R_f$-Wert 0,35. Die gefundenen $R_f$-Werte waren also im Vergleich mit dem Beispiel 20 genau umgekehrt.

Beispiel 22

Es wurde wie in Beispiel 20 verfahren mit dem Unterschied, daß die verwendete DC-Platte anstatt mit (2S, 2'RS)-N-(2'-Hydroxytetradecyl)-prolin mit (2S, 2'RS)-N-(2'-Hydroxyhexadecyl)-prolin als chiraler Selektor imprägniert war. In diesem Falle führte das (R)-Glutamin zu Flecken mit dem $R_f$-Wert 0,34 und das (S)-Glutamin zu Flecken mit dem $R_f$-Wert 0,19.

Beispiel 23

Das Beispiel 1 wurde wiederholt mit dem Unterschied, daß als zu untersuchende Probe ein Glutamin unbekannter Enantiomerenzusammensetzung und zu Vergleichszwecken (RS)-, (R)- und (S)-Glutamin eingesetzt und als Laufmittel ein Gemisch aus Methanol, Wasser, Acetonitril und Tetrahydrofuran im Volumenverhältnis 1 : 1 : 2 : 2 verwendet wurde. In diesem Falle führte das (R)-Glutamin zu Flecken mit dem $R_f$-Wert 0,70 und das (S)-Glutamin zu Flecken mit dem $R_f$-Wert 0,62.

Beispiel 24

Zum Zwecke der semiquantitativen Bestimmung von (R)-Glutamin in teilracemisiertem (S)-Glutamin durch visuellen Fleckengrößenvergleich wurde das Beispiel 1 wiederholt mit dem Unterschied, daß als zu untersuchende Probe ein teilracemisiertes (S)-Glutamin und zu Vergleichszwecken (RS)-, (R)- und (S)-Glutamin, sowie standardisierte 1 %-ige Glutamin-Lösungen mit 10, 8, 5, 4, 3, 2 und 1 % (R)-Glutamin, Rest jeweils (S)-Glutamin, eingesetzt wurden. Das (R)-Glutamin führte zu Flecken mit dem $R_f$-Wert 0,53 und das (S)-Glutamin zu Flecken mit dem $R_f$-Wert 0,37.

Durch visuellen Größenvergleich der Flecken mit dem $R_f$-Wert 0,53 konnte abgeschätzt werden, daß die zu untersuchende Probe etwa 1 % (R)-Glutamin enthielt.

Beispiel 25

Das Beispiel 1 wurde wiederholt mit dem Unterschied, daß als zu untersuchende Probe eine 3-Thiazolidin-4-carbonsäure unbekannter Enantiomerenzusammensetzung und zu Vergleichs-zwecken (RS)-, (R)- und (S)-3-Thiazolidin-4-carbonsäure eingesetzt wurden. In diesem Falle führte das (R)-Enantiomere zu Flecken mit dem $R_f$-Wert 0,52 und das (S)-Enatiomere zu Flecken mit dem $R_f$-Wert 0,42.

Diese Arbeitsweise läßt sich z. B. zum Nachweis von (S)-Cystein in teilracemisiertem (R)-Cystein heranziehen. Da sich die enantiomeren Cysteine als solche nach dem erfindungsgemäßen Verfahren nicht trennen lassen, werden sie zu den entsprechenden 3-Thiazolidin-4-carbonsäuren derivatisiert.

Zu diesem Zwecke wurden teilracemisiertes (R)-Cystein als zu untersuchende Probe und zu Vergleichszwecken (RS)-, (R)- und (S)-Cystein in einer Menge von jeweils 25 mg mit jeweils 25 mg

Paraformaldehyd vermischt, in einem Gemisch aus 2,5 ml Propan-2-ol und 25 µl konzentrierter Salzsäure gelöst und 2 Stunden auf 60 °C erwärmt. Nach dem Abkühlen auf 25 °C wurden die erhaltenen Reaktionsgemische in einer Menge von jeweils 2 µl direkt auf eine wie im Beispiel 1 beschrieben präparierte DC-Platte aufgetragen und mit dem in Beispiel 1 verwendeten Laufmittel entwickelt.

Nachdem die Laufmittelfront nach 45 Minuten eine Höhe von 14 cm erreicht hatte, wurde das Lösungsmittelgemisch verdampfen gelassen, die Platte mit der 0,1 %-igen Ninhydrin-Lösung besprüht und im Trockenschrank bei 125 °C getrocknet.

Das derivatisierte (R)-Cystein führte zu Flecken mit dem $R_f$-Wert 0,52 und das derivatisierte (S)-Cystein zu Flecken mit dem $R_f$-Wert 0,42.

Beispiel 26

Das Beispiel 25 wurde wiederholt mit dem einzigen Unterschied, daß anstatt der 3-Thiazolidin-4-carbonsäuren die entsprechenden 5,5-Dimethyl-3-thiazolidin-4-carbonsäuren eingesetzt wurden. In diesem Falle führte das (R)-Enantiomere zu Flecken mit dem $R_f$-Wert 0,50 und das (S)-Enantiomere zu Flecken mit dem $R_f$-Wert 0,35.

Diese Arbeitsweise läßt sich z. B. zur Trennung der Enantiomeren des Penicillamins heranziehen, wenn dieses vorher in der im Beispiel 21 für das Cystein beschriebenen Weise zu den entsprechenden 5,5-Dimethyl-3-thiazolidin-4-carbonsäuren derivatisiert und diese wie im Beispiel 25 weiterbehandelt werden. Das derivatisierte (R)-Penicillamin führt dann wieder zu Flecken mit dem $R_f$-Wert 0,50 und das derivatisierte (S)-Penicillamin wieder zu Flecken mit dem $R_f$-Wert 0,35.

Beispiele 27 bis 30

Mittels der in Beispiels 1 beschriebenen präparierten DC-Platte und des dort verwendeten Laufmittels wurden verschiedene racemische S-substituierte Cysteine getrennt. Die Auswertung ergab folgendes :

| Beispiel | (RS)-Cystein-Derivat | $R_f$-Werte |
|---|---|---|
| 27 | S-[(Methylmercapto)-methyl]-cystein | 0,37 und 0,49 |
| 28 | S-[2-(Methylmercapto)-ethyl]-cystein | 0,35 und 0,48 |
| 29 | S-(2-Chlorbenzyl)-cystein | 0,27 und 0,39 |
| 30 | S-[2-(2'-Pyridyl)-ethyl]-cystein | 0,19 und 0,31 |

Beispiel 31

Mittels der in Beispiel 1 beschriebenen präparierten DC-Platte und des dort verwendeten Laufmittels wurde racemisches O-(Benzyl)-serin getrennt. Die $R_f$-Werte der beiden Enantiomeren betrugen 0,26 und 0,38.

Beispiel 32

Mittels der in Beispiel 1 beschriebenen präparierten DC-Platte und des dort verwendeten Laufmittels wurde racemisches 3-Cyclopentyl-alanin getrennt. Die $R_f$-Werte der beiden Enantiomeren betrugen 0,29 und 0,38.

**Patentansprüche**

1. Verfahren zur dünnschichtchromatographischen Trennung von Enantiomeren der allgemeinen Formel

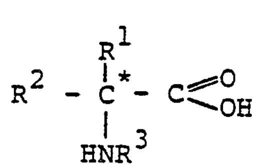

(I)

in der C* ein Asymmetriezentrum darstellt und die Substituenten $R^1$, $R^2$ und $R^3$ solche Bedeutungen haben, daß sie das Strukturelement

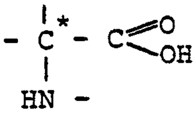

zu einer an sich bekannten α-Amino- oder α-Iminocarbonsäure oder einem Derivat einer solchen Carbonsäure ergänzen, welche bzw. welches keine freien Mercaptogruppen aufweist, mittels Ligandenaustausch an einer chiralen stationären Phase, dadurch gekennzeichnet, daß man die Trennung auf einer an sich bekannten, mit durch ein Silanderivat hydrophobiertem Kieselgel belegten Dünnschichtchromatographie-Platte vornimmt, die zusätzlich mit einer ionischen Verbindung eines zweiwertigen Übergangsmetalls und einem chiralen Selektor imprägniert ist, und als Laufmittel ein ternäres Gemisch aus einem mit Wasser mischbaren Alkanol, Wasser und Acetonitril oder ein quaternäres Gemisch aus einem mit Wasser mischbaren Alkanol, Wasser, Acetonitril und einem mit Wasser mischbaren cyclischen Ether einsetzt.

2. Verfahren nach Anspruch 1, dadurch gekennzeichnet, daß man als ionische Verbindung eines zweiwertigen Übergangsmetalls eine $Cu^{++}$-Verbindung einsetzt.

3. Verfahren nach Anspruch 1 oder 2, dadurch gekennzeichnet, daß man als chiralen Selektor ein optisch aktives Prolin-Derivat der allgemeinen Formel

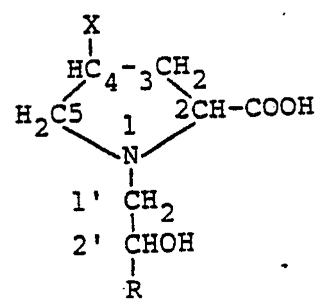

(II)

in der X Wasserstoff oder eine Hydroxygruppe und R einen unsubstituierten oder durch niedere Alkylgruppen substituierten Phenylrest oder einen geradkettigen oder verzweigten Alkylrest mit 1 bis 20 Kohlenstoffatomen bedeuten, einsetzt.

4. Verfahren nach einem der Ansprüche 1 bis 3, dadurch gekennzeichnet, daß man als mit Wasser mischbares Alkanol Methanol einsetzt.

5. Verfahren nach einem der Ansprüche 1 bis 4, dadurch gekennzeichnet, daß man als mit Wasser mischbaren cyclischen Ether Tetrahydrofuran einsetzt.

**Claims**

1. A process for the separation by thin layer chromatography of enantiomers corresponding to the general formula

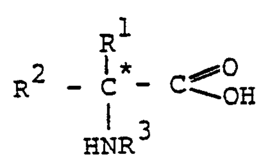

(I)

in which C* represents a centre of asymmetry and the substituents $R^1$, $R^2$ and $R^3$ have meanings such that they make up the structural element

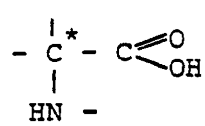

to a known α-amino or α-imino carboxylic acid or a derivative of such a carboxylic acid which has no free mercapto groups by exchange of ligands at a chiral stationary phase, characterised in that separation is carried out on a known thin layer chromatographic plate covered with silica gel which has been rendered hydrophobic by a silane derivative, the plate additionally being impregnated with an ionic

compound of a divalent transition metal and a chiral selector, and in that a ternary mixture of a water-miscible alkanol, water and acetonitrile or a quaternary mixture of a water-miscible alkanol, water, acetonitrile and a water-miscible cyclic ether is used as eluant.

2. A process according to claim 1, characterised in that a $Cu^{++}$ compound is used as ionic compound of a divalent transition metal.

3. A process according to claim 1 or 2, characterised in that, an optically active proline derivative corresponding to the general formula

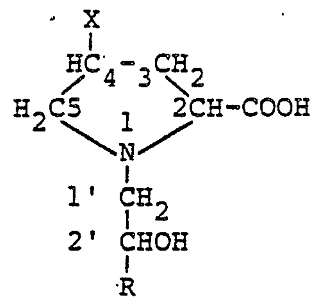

(II)

in which X represents hydrogen or a hydroxy group and R represents an unsubstituted phenyl radical or a phenyl radical substituted by lower alkyl groups or a straight-chained or branched alkyl radical containing from 1 to 20 carbon atoms, is used as a chiral selector.

4. A process according to one of claims 1 to 3, characterised in that methanol is used as water-miscible alkanol.

5. A process according to one of claims 1 to 4, characterised in that tetrahydrofuran is used as water-miscible cyclic ether.

**Revendications**

1. Procédé pour la séparation, par chromatographie en couche mince, d'énantiomères de formule générale :

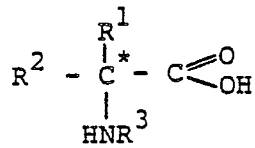

(I)

dans laquelle C* représente un centre d'asymétrie et les substituants $R^1$, $R^2$ et $R^3$ ont des significations telles qu'ils complètent l'élément structural :

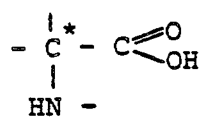

en un acide α-amino- ou α-iminocarboxylique connu en lui-même ou en un dérivé d'un tel acide carboxylique, ne comportant pas de groupes mercapto libres, par échange de ligands au niveau d'une phase stationnaire chirale, caractérisé en ce que l'on effectue la séparation sur une plaque pour chromatographie en couche mince connue en elle-même, revêtue d'un gel de silice, rendue hydrophobe au moyen d'un dérivé silane, qui est en outre imprégnée d'un composé ionique d'un métal de transition divalent, ainsi que d'un sélecteur chiral, et on utilise en tant que solvant mobile un mélange ternaire composé d'un alcanol miscible à l'eau, d'eau et d'acétonitrile, ou un mélange quaternaire composé d'un alcanol miscible à l'eau, d'eau, d'acétonitrile et d'un éther cyclique miscible à l'eau.

2. Procédé selon la revendication 1, caractérisé en ce que l'on utilise un composé cuivrique comme composé ionique d'un métal de transition divalent.

3. Procédé selon la revendication 1 ou 2, caractérisé en ce que l'on utilise en tant que sélecteur chiral un dérivé optiquement actif de la proline, de formule générale :

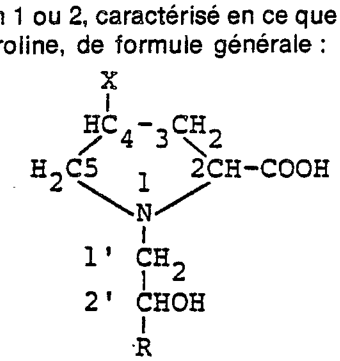

(II)

dans laquelle X représente un atome d'hydrogène ou un groupe hydroxyle, et R représente un radical phényle non substitué ou substitué par des groupes alkyles inférieurs ou un radical alkyle à chaîne droite ou ramifiée ayant de 1 à 20 atomes de carbone.

4. Procédé selon l'une quelconque des revendications 1 à 3, caractérisé en ce que l'on utilise, en tant qu'alcanol miscible à l'eau, le méthanol.

5. Procédé selon l'une quelconque des revendications 1 à 4, caractérisé en ce que l'on utilise en tant qu'éther cyclique miscible à l'eau, le tétrahydrofuranne.